Europäisches Patentamt

European Patent Office

Office européen des brevets

(11) Veröffentlichungsnummer: **0 185 252**
**B1**

(12)                    **EUROPÄISCHE PATENTSCHRIFT**

(45) Veröffentlichungstag der Patentschrift:
**11.10.89**

(51) Int. Cl.⁴: **C 07 C  7/04, F 25 J  3/08**

(21) Anmeldenummer: **85115406.2**

(22) Anmeldetag: **04.12.85**

(54) Verfahren zur Gewinnung von C2+- oder von C3+-Kohlenwasserstoffen.

(30) Priorität: **17.12.84 DE 3445994**

(43) Veröffentlichungstag der Anmeldung:
**25.06.86 Patentblatt 86/26**

(45) Bekanntmachung des Hinweises auf die Patenterteilung: **11.10.89 Patentblatt 89/41**

(84) Benannte Vertragsstaaten:
**DE FR GB IT NL SE**

(56) Entgegenhaltungen:
**DE—A— 3 408 760**
**GB—A— 2 102 931**
**US—A— 2 966 402**

(73) Patentinhaber: **Linde Aktiengesellschaft**
**Abraham-Lincoln-Strasse 21**
**D-6200 Wiesbaden (DE)**

(72) Erfinder: **Bauer, Heinz, Dr. rer. nat.**
**Nibelungenstrasse 6**
**D-8000 München 19 (DE)**

(74) Vertreter: **Schaefer, Gerhard, Dr. et al**
**Linde Aktiengesellschaft Zentrale Patentabteilung**
**D-8023 Höllriegelskreuth (DE)**

## Beschreibung

Die Erfindung betrifft ein Verfahren zur Abtrennung von $C_{2+}$- oder $C_{3+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom abgekühlt, partiell kondensiert und in einem Abscheider in eine flüssige und eine gasförmige Fraktion getrennt wird, bei dem die gasförmige Fraktion arbeitsleistend entspannt und die flüssige Fraktion durch Rektifikation in einen $C_{2+}$- bzw. $C_{3+}$-Kohlenwasserstoffe enthaltenden Produktstrom und einen überwiegend leichter siedende Komponenten enthaltenden Restgasstrom zerlegt wird und der Restgasstrom dem Gasstrom vor dessen Entspannung zugemischt wird.

Derartige Verfahren dienen vor allem zur Abtrennung von Ethan oder von Propan aus Erdgasen oder anderen Gasen, beispielsweise Raffinerieabgasen. Außerdem eignen sich diese Verfahren zur Abtrennung von vergleichbaren ungesättigten Kohlenwasserstoffen, also beispielsweise von Ethylen oder Propylen, sofern diese Komponenten im zu zerlegenden Gasgemisch enthalten sind, was beispeilsweise bei Raffinerieabgasen der Fall sein kann. Die Weiterverarbeitung von Raffinerieabgasen ist in letzter Zeit wirtschaftlich interessant geworden, da die Marktpreise für LPG ($C_3/C_4$-Kohlenwasserstoffgemisch) gestiegen sind, während umgekehrt Vakuumrückstände sowie Schweröl schlecht verkäuflich sind. Aus diesem Grund werden schlecht vermarktbare schwere Produkte zur Deckung des internen Brennstoffbedarfs einer Raffinerie verfeuert, während gut verkäufliche $C_{3+}$-Kohlenwasserstoffe aus dem Abgas, das insbesondere bei der Verarbeitung von leichten Rohölbestandteilen zu Benzin in großen Mengen anfällt, abgetrennt werden.

In der GB-A-2 102 931 ist bereits ein Verfahren der eingangs genannten Art beschrieben, bei dem kondensierbare Kohlenwasserstoffe aus einem Gas abgetrennt werden. Aus einer Rektifikation, bei der ein schwere Kohlenwasserstoffe enthaltender Produktgasstrom gewonnen wird, wird dort ein Restgasstrom entnommen und mit einer unter erhöhtem Druck stehenden gasförmigen Fraktion, die aus einem Abscheider entnommen wird und weiterzerlegt werden soll, vermischt. Das Gemisch wird einer weiteren Phasentrennung unterworfen. Die dabei entstehende gasförmige Fraktion wird arbeitsleistend entspannt, die flüssige Fraktion nach Vermischung mit der entspannten gasförmigen Fraktion und nach einer nochmaligen Phasentrennung zur Rektifikation geführt.

Die Rückführung des Gases vom Kopf der Rektifikation ist an sich günstig, da somit für die arbeitsleistende Entspannung eine größere Durchsatzmenge an Gas zur Verfügung steht, wodurch mehr Kälte erzeugt werden kann. Die gasförmige Fraktion, die aus dem Abscheider entnommen wird, liegt am Taupunkt vor, ebenso wie der Restgasstrom aus dem Kopf der Rektifikation. Bei der Mischung dieser beiden jeweils am Taupunkt befindlichen Fraktionen tritt im allgemeinen eine Kondensatbildung ein. Beim Mischen zusätzlich auskondensierte Bestandteile müssen vor dem Entspannen abgetrennt werden, um einen sicheren Betrieb einer Expansionsturbine zu gewährleisten. Dazu muß eine weitere Phasentrennung durchgeführt werden, für die ein zusätzlicher Abscheider notwendig ist.

Der Erfindung liegt deshalb die Aufgabe zugrunde, ein Verfahren der eingangs genannten Art so auszugestalten, daß die Abtrennung von $C_{2+}$- bzw. $C_{3+}$-Kohlenwasserstoffen mit verringertem Aufwand ermöglicht wird.

Diese Aufgabe wird dadurch gelöst, daß der Restgasstrom in den Abscheider für die Abtrennung der gasförmigen Fraktion aus dem partiell kondensierten Rohgas geleitet und dort mit der nach der partiellen Kondensation gasförmig verbleibenden Fraktion des Rohgases vermischt wird und dieses Gemisch der arbeitsleistenden Entspannung unterworfen wird.

Beim erfindungsgemäßen Verfahren findet die Vermischung zwischen gasförmiger Fraktion und Restgasfraktion innerhalb des Abscheiders statt, der ohnehin für die Phasentrennung bei dem partiell kondensierten Rohgasstrom benötigt wird. Die durch die Vermischung zusätzlich auskondensierenden Komponenten bewegen sich im Abscheider nach unten und werden mit der flüssigen Fraktion zur Rektifikation geführt. Somit genügt ein einziger Abscheider, um sowohl die bei der partiellen Kondensation entstandenen als auch die bei der Vermischung gebildeten flüssigen Bestandteile vom gasförmigen Anteil abzutrennen. Die Verfahrensführung wird dadurch vereinfacht, der apparative Aufwand wird gering gehalten.

Sofern die Rektifikation bei einem höheren Druck als dem Druck des partiell kondensierten Gasstroms erfolgt, wird in Weiterbildung des erfindungsgemäßen Verfahrens die flüssige Fraktion auf den Druck der Rektifikation gepumpt und das bei der Rektifikation gebildete Restgas in den Abscheider für die Trennung der flüssigen von der gasförmigen Fraktion entspannt.

Diese Verfahrensführung erweist sich als besonders günstig, da einerseits keine Verdichtung des Gasstroms selbst, sondern nur das sehr viel weniger aufwendige Pumpen der flüssigen Fraktion auf den höheren Rektifikationsdruck erforderlich ist, und andererseits das Entspannen des Restgases der Rektifikation in den Rohgasabscheider auf einfache Weise die Abtrennung des bei der Mischung anfallenden Kondensats ermöglicht, ohne daß hierzu weitere Bauteile erforderlich wären.

Zur Vermeidung der Kondensatbildung bei der Mischung kann andererseits auch der Restgasstrom oder das Gemisch soweit angewärmt werden, daß die Gemischtemperatur nicht unterhalb des Taupunkts liegt. Die Anwärmung kann beispielsweise gegen abzukühlendes Rohgas durchgeführt werden.

Sofern die Rektifikation bei einem niedrigeren Druck als dem Druck des partiell kondensierten Gasstroms durchgeführt wird, ist erfindungsgemäß vorgesehen, daß der partiell kondensierte Gasstrom auf den Druck des Restgasstroms entspannt wird, bevor die Vermischung stattfindet.

Bei der Verarbeitung von Gasströmen, die reich an leichter als Methan siedenden Komponenten sind, ist in Weiterbildung der Erfindung vorgesehen, daß eine Anreicherung dieser Komponenten erfolgen kann, wozu vor der arbeitsleistenden Entspannung der gasförmigen Fraktion aus dieser $C_1$- und $C_2$-Kohlenwasserstoffe durch partielle Kondensation abgetrennt werden. Diese Verfahrensweise kann beispielsweise bei der Abtrennung von $C_{2+}$- bzw. $C_{3+}$-Kohlenwasserstoffen und von Stickstoff aus stickstoffreichem Erdgas oder insbesondere zur Gewinnung der genannten schweren Kohlenwasserstoffe und von Wasserstoff aus wasserstoffreichen Raffinerieabgasen zum Einsatz kommen. Eine solche Abtrennung ist insbesondere dann von Vorteil, wenn der Einsatzstrom einen relativ hohen Anteil tiefsiedender Komponenten aufweist, beispielsweise einen Wasserstoffgehalt in der Größenordnung von 50 bis 90 %. Eine solche Wasserstoffmenge reicht nämlich aus, die für die zusätzliche Abtrennung benötigte Kälte bei der Entspannung zu erzeugen, ohne daß zusätzlich Fremdenergie aufgewendet werden muß.

In vielen Anwendungsfällen ist eine weitere Zerlegung des $C_{2+}$- bzw. $C_{3+}$-Kohlenwasserstoffprodukts, insbesondere eine Trennung zwischen einem $C_3/C_4$-Kohlenwasserstoffgemisch und $C_{5+}$-Kohlenwasserstoffen erwünscht. Zu diesem Zweck wird gemäß einer bevorzugten Ausgestaltung des erfindungsgemäßen Verfahrens vor der Bildung der flüssigen und der gasförmigen Fraktion ein Großteil der $C_{5+}$-Kohlenwasserstoffe aus dem Gasstrom abgetrennt, sofern die Konzentration dieser Bestandteile so groß ist, daß sich eine derartige Abtrennung lohnt.

Die $C_{5+}$-Abtrennung erfolgt zweckmäßig durch partielle Kondensation bei einer Temperatur oberhalb der Temperatur, bei der die oben erwähnte flüssige und gasförmige Fraktionen gebildet werden. Durch die vorherige Abtrennung der schweren Komponenten ist das der Rektifikation zugeführte Gemisch nahezu frei an $C_{5+}$-Kohlenwasserstoffen, so daß bei der nachfolgenden Rektifikation der flüssigen Fraktion ein Produktstrom gewonnen wird, der bei einer $C_{3+}$-Abtrennung eine handelsübliche LPG-Fraktion bildet.

Um die Ausbeute von $C_3$- und $C_4$-Kohlenwasserstoffen zu erhöhen, wird in weiterer Ausbildung dieser Verfahrensvariante vorgeschlagen, daß die abgetrennten schweren Kohlenwasserstoffe ebenfalls der Rektifikation zugeführt werden, wobei die Einspeisung der $C_{5+}$-Fraktion in eine Rektifiziersäule entsprechend dem Gleichgewichtsverlauf in der Rektifiziersäule unterhalb der Einspeisung der bei der partiellen Kondensation gebildeten flüssigen Fraktion erfolgt und wobei ferner vorgesehen ist, daß ein im wesentlichen $C_3$- und $C_4$-Kohlenwasserstoffe enthaltender Strom zwischen den beiden Einspeisungen entnommen wird. Durch die zusätzliche Rektifikation der $C_{5+}$-Fraktion werden auch noch der $C_3/C_4$-Kohlenwasserstoffe, die bei der Bildung der $C_{5+}$-Fraktion durch partielle Kondensation in Lösung gegangen sind, als Produkt zurückgewonnen. Zwischen den beiden Einspeisestellen bildet innerhalb der Rektifiziersäule ein Bereich maximaler $C_{3/4}$-Konzentration aus, wo in günstiger Weise der $C_3/C_4$-Produktstrom entnommen wird.

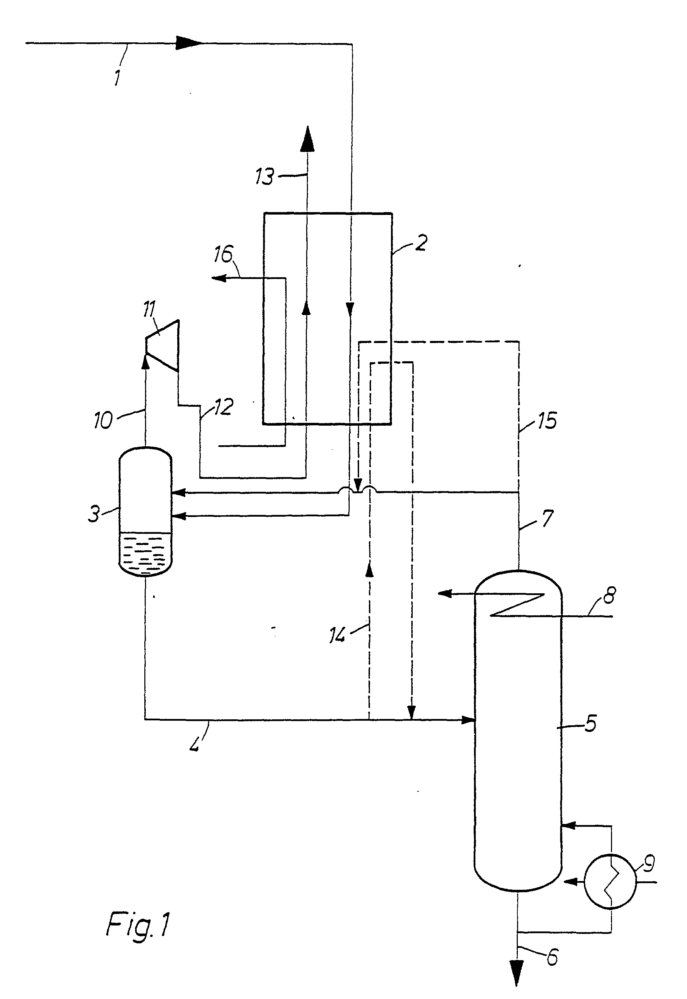

Weitere Einzelheiten der Erfindung werden nachfolgend anhand des in der Zeichnung schematisch dargestellten Ausführungsbeispiels erläutert. Figur 1 zeigt eine Prinzipskizze des erfindungsgemäßen Verfahrens.

Bei diesem Ausführungsbeispiel wird über Leitung 1 der zu zerlegende Gasstrom unter erhöhtem Druck und bei annähernd Umgebungstemperatur einem Wärmetauscher 2 zugeführt, in dem er so weit abgekühlt wird, daß der größte Teil der abzutrennenden Kohlenwasserstoffe, also der $C_{2+}$- bzw. der $C_{3+}$-Kohlenwasserstoffe kondensiert ist. Der partiell kondensierte Gasstrom wird in einem Abscheider 3 einer Phasentrennung unterzogen, wobei das Kondensat über Leitung 4 einer Rektifiziersäule 5 zugeleitet wird, in der es in eine $C_{2+}$- bzw. $C_{3+}$-Fraktion, die als Produktstrom über Leitung 6 aus dem Sumpf der Säule abgezogen wird, und in einen leichter siedende Anteile enthaltenden Restgasstrom 7 zerlegt wird. Die Rektifikation wird durchgeführt unter Verwendung einer mit Fremdkälte betriebenen Kopfkühlung 8 sowie einer beispielsweise mit Niederdruckdampf beheizten Sumpfheizung 9, in der ein von Leitung 6 abgezweigter Teilstrom des Sumpfprodukts aufgeheizt und dann wieder in den Säulensumpf zurückgeführt wird.

Das über Leitung 7 abgezogene Kopfprodukt der Rektifikation, das im wesentlichen aus Komponenten besteht, die leichter als die über Leitung 6 abgezogene Produktfraktion sieden, wird erfindungsgemäß in den Abscheider 3 geleitet und dort mit der nach der partiellen Kondensation gasförmig verbliebenen Fraktion des Rohgases vermischt. Bei der Vermischung gegebenenfalls auftretendes Kondensat wird im Abscheider 3 abgetrennt, so daß über Leitung 10 ein flüssigkeitsfreies Gasgemisch abgezogen und der Expansionsturbine 11 zugeführt wird. Nach der arbeitsleistenden Entspannung in der Turbine 11 tritt das Turbinenabgas über Leitung 12 in den Wärmetauscher 2 ein und gibt seinen Kälteinhalt an das zu zerlegende Gasgemisch ab. Es wird schließlich über Leitung 13 als leichte Fraktion abgegeben und kann beispielsweise als Heizgas verwendet werden.

Die über die Leitungen 4 und 10 aus dem Abscheider 3 bzw. über Leitung 7 aus der Rektifiziersäule 5 abgezogenen Ströme können vor ihrer weiteren Verarbeitung gegebenenfalls auf geeignete Temperaturniveaus angewärmt bzw. abgekühlt werden, was beispielsweise im Wärmetauscher 2 erfolgen kann. Für die Ströme in den Leitungen 4 und 7 ist dies durch gestrichelte Linien 14 bzw. 15 angedeutet. Der Fremdkältebedarf für das Verfahren wird durch einen Kältekreislauf gedeckt, der durch 16 schematisch angedeutet ist und die Kühlung des Gasgemisches im Wärmetauscher 2 gemeinsam mit anzuwärmenden Verfahrensströmen bewirkt.

## Patentansprüche

1. Verfahren zur Abtrennung von C$_{2+}$- oder von C$_{3+}$-Kohlenwasserstoffen aus einem leichte Kohlenwasserstoffe und gegebenenfalls leichter als Methan siedende Komponenten enthaltenden Gasstrom (1), bei dem der unter erhöhtem Druck stehende Gasstrom abgekühlt, partiell kondensiert und in einem Abscheider (3, 28) in eine flüssige und eine gasförmige Fraktion getrennt wird, bei dem die gasförmige Fraktion arbeitsleistend entspannt und die flüssige Fraktion durch Rektifikation (5, 29) in einen C$_{2+}$- bzw. C$_{3+}$-Kohlenwasserstoffe enthaltenden Produktstrom (6, 31) und einen überwiegend leichter siedende Komponenten enthaltenden Restgasstrom (7) zerlegt wird und der Restgasstrom dem Gasstrom vor dessen Entspannung zugemischt wird, dadurch gekennzeichnet, daß der Restgasstrom (7) in den Abscheider (3, 28) geleitet und dort mit der nach der partiellen Kondensation gasförmig verbleibenden Fraktion des Rohgases vermischt wird und dieses Gemisch (10) der arbeitsleistenden Entspannung (11) unterworfen wird.

2. Verfahren nach Anspruch 1, dadurch gekennzeichnet, daß die Rektifikation (5) bei einem höheren Druck als dem Druck des partiell kondensierten Gasstromes erfolgt, wobei die flüssige Fraktion (4) auf den Druck der Rektifikation gepumpt (21) und das bei der Rektifikation (5) gebildete Restgas (7) in den Abscheider (3) für die Trennung der flüssigen von der gasförmigen Fraktion entspannt wird (22).

3. Verfahren nach Anspruch 1, dadurch gekennzeichnet, daß der Restgasstrom (7) und/oder das Gemisch (10) vor der arbeitsleistenden Entspannung (11) mindestens soweit angewärmt wird, daß die Gemischtemperatur nicht unterhalb des Taupunktes liegt.

4. Verfahren nach Anspruch 3, dadurch gekennzeichnet, daß die Rektifikation bei einem niedrigeren Druck als dem Druck des partiell kondensierten Gasstromes erfolgt, wobei der partiell kondensierte Gasstrom auf den Druck des Restgasstromes entspannt wird.

5. Verfahren nach einem der Ansprüche 1 bis 4, dadurch gekennzeichnet, daß die flüssige Fraktion (4) vor der Rektifikation (5, 29) mindestens teilweise gegen den abzukühlenden Gasstrom (1) erwärmt wird.

6. Verfahren nach einem der Ansprüche 1 bis 5, dadurch gekennzeichnet, daß bei der Verarbeitung eines Gasstroms, der reich an leichter als Methan siedenden Komponenten ist, vor der arbeitsleistenden Entspannung der gasförmigen Fraktion aus dieser C$_1$- und C$_2$-Kohlenwasserstoffe durch partielle Kondensation abgetrennt werden.

7. Verfahren nach einem der Ansprüche 1 bis 6, dadurch gekennzeichnet, daß vor der Bildung der flüssigen und der gasförmigen Fraktion ein Großteil von gegebenenfalls im Gasstrom enthaltenen C$_{5+}$-Kohlenwasserstoffen abgetrennt wird.

8. Verfahren nach Anspruch 7, dadurch gekennzeichnet, daß die abgetrennten C$_{5+}$-Kohlenwasserstoffe (25) ebenfalls der Rektifikation zugeführt werden, daß die Einspeisung in eine Rektifiziersäule (29) unterhalb der Einspeisung der bei der partiellen Kondensation gebildeten flüssigen Fraktion (4) erfolgt und daß ein im wesentlichen C$_3$- und C$_4$-Kohlenwasszerstoffe enthaltender Strom (30) zwischen den beiden Einspeisungen entnommen wird.

## Claims

1. A process for separating C$_{2+}$- or C$_{3+}$-hydrocarbons from a gas stream (1) containing light hydrocarbons and, in case, components which have a lower boiling point than methane, wherein the gas stream under increased pressure is cooled, partially condensed and separated in a separator (3, 28) into a liquid fraction and a gaseous fraction, wherein the gaseous fraction is expanded with the production of external work and the liquid fraction is split by rectification (5, 29) into a product stream (6, 31) containing C$_{2+}$- or C$_{3+}$-hydrocarbons and into a residual gas stream (7) containing mainly lower boiling components and the residual gas stream is admixed to the gas stream prior to its expansion, characterised in that the residual gas stream (7) is fed into the separator (3, 28) where it is mixed with that fraction of the cooled gas which remains in gaseous form after the partial condensation and this mixture (10) is subjected to the work producing expansion (11).

2. A process as claimed in claim 1, characterised in that the rectification (5) is carried out at a higher pressure than the pressure of the partially condensed gas stream, where the liquid fraction (4) is pumped (21) to the pressure of the rectification and the residual gas (7) formed by the rectification (5) is expanded (22) in the separator (3) in order to separate the liquid fraction from the gaseous fraction.

3. A process as claimed in claim 1, characterised in that prior to the work producing expansion (11) the residual gas stream (7) and/or the mixture (10) is heated at least to the extent that the temperature of the mixture is not below the dew point.

4. A process as claimed in claim 3, characterised in that the rectification is carried out at a lower pressure than the pressure of the partially condensed gas stream, where the partially condensed gas stream is expanded to the pressure of the residual gas stream.

5. A process as claimed in one of the claims 1 to 4, characterised in that prior to the rectification (5, 29) the liquid fraction (4) is heated at least partially in the presence of the gas stream (1) which is to be cooled.

6. A process as claimed in one of the claims 1 to 5, characterised in that for the processing of a gas stream which is rich in components which have a lower boiling point than methane, prior to the work producing expansion of the gaseous fraction, $C_1$- and $C_2$-hydrocarbons are separated from the gaseous fraction by partial condensation.

7. A process as claimed in one of the claims 1 to 6, characterised in that prior to the formation of the liquid fraction and the gaseous fraction, the majority of any $C_{5+}$-hydrocarbons contained in the gas stream are separated.

8. A process as claimed in claim 7, characterised in that the separated $C_{5+}$-hydrocarbons (25) are likewise supplied to the rectification stage, that the input into a rectification column (29) takes place below the imput point of the liquid fraction (4) formed during the partial condensation and that a stream (30) fundamentally containing $C_3$- and $C_4$-hydrocarbons is withdrawn between the two input points.

## Revendications

1. Procédé pour séparer des hydrocarbures en $C_{2+}$ ou $C_{3+}$ à partir d'un courant gazeux (1), contenant des hydrocarbures légers et éventuellement des composants dont le point d'ébullition est inférieur à celui du méthane, procédé dans lequel le courant gazeux, sous pression élevée, est refroidi, partiellement condensé et, dans un séparateur (3, 28), séparé en une fraction liquide et une fraction gazeuse, la fraction gazeuse subissant une détente génératrice de travail, et la fraction liquide étant décomposée, par rectification (5, 29), en un courant de produits (6, 31) contenant des hydrocarbures en $C_{2+}$ ou $C_{3+}$ et en un courant gazeux résiduel (7) contenant essentiellement des composants à point d'ébullition plus faible, le courant gazeux résiduel étant mélangé au courant gazeux avant cette détente, ce procédé étant caractérisé en ce que le courant gazeux résiduel (7) est envoyé dans le séparateur (3, 28) et y est mélangé à la fraction du gaz brut restant sous forme gazeuse après la condensation partielle, et que ce mélange (10) est soumis à la détente (11) fournissant du travail.

2. Procédé selon la revendication 1, caractérisé en ce que la rectification (5) est mise en œuvre sous une pression supérieure à la pression du courant gazeux partiellement condensé, la fraction liquide (4) étant amenée par pompe (21) à la pression de rectification, et le gaz résiduel (7), formé lors de la rectification (5), étant détendu dans le séparateur (3) pour assurer la séparation de la fraction liquide d'avec la fraction gazeuse.

3. Procédé selon la revendication 1, caractérisé en ce que le courant gazeux résiduel (7) et/ou le mélange (10) est, avant la détente génératrice de travail (11), chauffée au moins jusqu'à ce que la température du mélange ne soit pas inférieur au point de rosée.

4. Procédé selon la revendication 3, caractérisé en ce que la rectification est mise en œuvre sous une pression inférieure à la pression du courant gazeux partiellement condensé, le courant gazeux partiellement condensé étant détendu jusqu'à la pression du courant gazeux résiduel.

5. Procédé selon l'une des revendications 1 à 4, caractérisé en ce que la fraction liquide (4) est, avant la rectification (5, 29), chauffée au moins en partie par échange avec le courant gazeux (1) à refroidir.

6. Procédé selon l'une des revendications 1 à 5, caractérisé en ce que, lors du traitement d'un courant gazeux riche en composants à point d'ébullition inférieur à celui du méthane, la fraction gazeuse est, avant la détente génératrice du travail, séparée de ces hydrocarbures en $C_1$ et $C_2$ par condensation partielle.

7. Procédé selon l'une des revendications 1 à 6, caractérisé en ce qu'une part importante d'hydrocarbures en $C_{5+}$ éventuellement contenus dans le courant gazeux sont séparés avant la formation de la fraction liquide et de la fraction gazeuse.

8. Procédé selon la revendication 7, caractérisé en ce que les hydrocarbures en $C_{5+}$ (25) sont eux aussi envoyés à la rectification, que l'injection dans une colonne de rectification (29) a lieu endessous du point d'injection de la fraction liquide (4) formée par la condensation partielle, et que l'on soutire entre ces deux points d'injection un courant (30) contenant pour l'essentiel des hydrocarbures en $C_3$ et $C_4$.

Fig.1